# EUROPEAN PATENT SPECIFICATION

(11) **EP 0 724 586 B1**
(45) Date of publication and mention of the grant of the patent: **23.01.2002**
(21) Application number: 94930057.8
(22) Date of filing: 05.10.1994
(51) Int. Cl.: C07D 487/04

(54) **PROCESS FOR PREPARING N-(2,6-DICHLORO-3-METHYLPHENYL)-5-7-DIHALO[1,2,4]TRIAZOLO[1,5A]PYRIMIDINE-2-SULFONAMIDE BY CYCLIZATION AND HALO-DEHYDROXYLATION**
VERFAHREN ZUR HERSTELLUNG VON N-(2,6-DICHLORO-3-METHYLPHENYL)-5-7-DIHALO[1,2,4]TRIAZOLO[1,5A]PYRIMIDIN-2-SULFONAMID DURCH CYCLISIERUNG UND HALO-DEHYDROXYLIERUNG
PROCEDE DE PREPARATION DE N-(2,6-DICHLORO-3-METHYLPHENYL)-5-7-DIHALO[1,2,4]TRIAZOLO[1,5A]PYRIMIDINE-2-SULFONAMIDE PAR CYCLISATION ET HALO-DESHYDROXYLATION

(30) Priority: 18.10.1993 US 138560
(43) Date of publication of application: 07.08.1996
(73) Proprietor: Dow AgroSciences LLC, Indianapolis, Indiana 46268 (US)
(72) Inventor: RINGER, James W., Midland, MI 48642 (US); BUDD, Jeffrey T., Midland, MI 48642 (US); TOBEY, Richard E., Midland, MI 48640 (US); ZETTLER, Mark W., Midland, MI 48640 (US)
(74) Representative: Raynor, John
(86) International application number: US9411333
(87) International publication number: WO9511246

(56) References cited:
- EP-A- 0 444 747
- E. MÜLLER (EDITOR) 'Methoden der Organischen Chemie, Band VIII, Sauerstoffverbindungen III, pages 464 - 469' 1952 , GEORG THIEME VERLAG , STUTTGART DE see page 464, line 9 - page 466, last paragraph

## Description

### Field of the Invention

The invention relates to a process for preparing an intermediate which could be used for the production of an agricultural product, such as a herbicide, fungicide or insecticide. More specifically, the invention relates to an improved process for making N-(2,6-dichloro-3-methylphenyl), (4-bromo-2,6 dichloro-3-methylphenyl), (2,6-dibromo-3-methylphenyl), (2,4-dibromo-3-methylphenyl), (4,6-dibromo-3-methylphenyl), or (4-bromo-3-methylphenyl),-5-7-dihalo[1,2,4]triazolo[1,5a]pyrimidine-2-sulfonamide (which are all hereinafter referred to as DCSA).

### Background of the Invention

DCSA is an agricultural intermediate used to produce agricultural herbicides, pesticides and the like. Typically, DCSA is made by cyclizing an amino triazole with dimethylmalonate and sodium methoxide to form a 5,7-dihydroxy pyrimidine intermediate. The pyrimidine intermediate is isolated by filtration, aqueous washing and drying, but these steps lead to partial decomposition of the pyrimidine ring. The pyrimidine intermediate is further converted to DCSA with phosphorus oxychloride. The problem with this method is that the yields in both reactions are very low, the method is unreliable and has two aqueous to anhydrous solvent changes. An additional problem with this method is that the DCSA is isolated by distillation of the phosphorus oxychloride to a tar residue that is followed by an aqueous work-up and filtration.

Another method of making DCSA is to treat an amino triazole with malonyl chloride in acetonitrile to form 5,7 dihydroxy pyrimidine intermediate and to dry it. The residues are then dissolved in phosphorus oxychloride and heated to 90°C for greater than 24 hours. Another technique is to treat an aminotriazole with an acid, such as malonic, in phosphorus oxychloride and heat the mixture to 90°C for greater than 24 hours. In both reactions, after the unreacted phosphorus oxychloride is evacuated, the resulting mixture contains tars and is quenched with water to isolate the 5,7 dihalo pyrimidine. This method is preferred to the previous method discussed above, but is still disadvantageous because malonic acid decomposes under high temperatures, high phosphorus waste streams are created, and the resulting desired compounds are of low purity.

European Patent Application No. 0444747 discloses a method of forming DCSA by reacting an amino triazole derivative with malonyl halide in the presence of phosphorus oxyhalide. The reaction temperature is low e.g., preferably from 20°C to 50°C and the reaction times are generally short e.g., usually from 8 to 24 hours. However, the yields obtained are generally low e.g. ~30%.

It is highly desirable to have a process that does not create phosphorus waste streams, uses catalysts and solvents that are easy and economical to handle from a manufacturing, environmental and cost perspective and that results in high yields of the desired products.

### Summary of the invention

According to a first aspect of the present invention there is provided a process for preparing DCSA, a compound of formula I 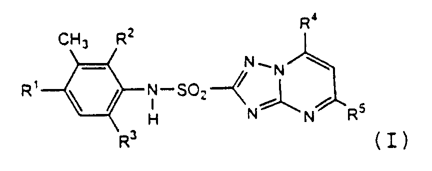 where R⁴ and R⁵ are both Cl or both Br, and the values of R¹, R² and R³ have one of the following combinations:

| R¹ | R² | R³ |
|---|---|---|
| H | Cl | Cl |
| Br | Cl | Cl |
| H | Br | Br |
| Br | Br | H |
| Br | H | Br |
| Br | H | H |

which comprises combining DCM-ATSA, a compound of the formula II 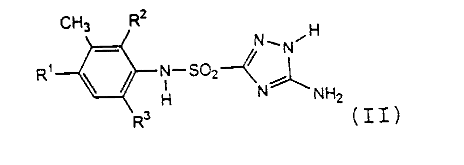 wherein R¹, R², and R³ are as defined in formula I, with malonic acid, in the presence of either phosphorus pentachloride and a phosphorous oxychloride solvent when a product wherein R⁴ and R⁵ are Cl is desired or phosphorus pentabromide and a phosphorus oxybromide solvent when a product wherein R⁴ and R⁵ are Br is desired at a temperature between 0°C and 60°C, to form a phosphorus tetrachloride or phosphorus tetrabromide adduct of the compound of formula II (DCM-ATSA-PCl₄), and
cyclising the phosphorus tetrachloride or phophorus tetrabromide adduct of the compound of formula II (DCM-ATSA-PCl₄) with the malonic acid, to produce a mixture of DHSA, a compound of formula III 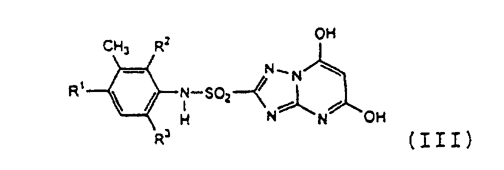 wherein R¹, R² and R³ are as defined in formula I, and a mixture of isomeric compounds of formulas IVa and Ivb, known as HCSA, 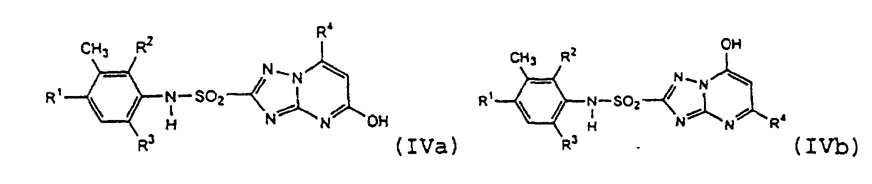 wherein R¹, R², R³ and R⁴ are as defined in formula I, and, after cyclization is complete,
halodehydroxylating the mixture of compounds of formulas III, IVa and IVb to form the product of formula I.

The invention relates to a process for making DCSA by cylizing DCM-ATSA with an acid and phosphorous pentachloride or pentabromide in the presence of a solvent to produce two isomeric compounds known as HCSA and DHSA. The resulting intermediates from the cyclisation are converted by a halo-dehydroxylation reaction to produce DCSA.

Prior to cyclisation reaction of DCM-ATSA, phosphorus pentachloride or pentabromide is made in-situ. Phosphorus pentachloride is made by reacting phosphorus trichloride and chlorine gas. The solvent in the reaction is typically phosphorous oxychloride.

The reaction is exothermic, but the temperature of the reaction is usually maintained between about -20°C and about 80°C.

After the phosphorous pentachloride is formed, the reactor is charged with DCM-ATSA and an acid. The DCM-ATSA and acid additions are exothermic, so the temperature is controlled between about 0°C to about 60°C. It is thought that DCM-ATSA and phosphorous pentachloride react to form a DCM-ATSA-PCl₄ adduct which further reacts with the acid. After the acid is added to the reactor, the DCM-ASTA-PCl₄ intermediate is cyclized with the acid under cool conditions to produce DHSA and HCSA intermediates. The conversion of DHSA and HCSA to DCSA by the halo-dehydroxylation reaction with phosphorous oxychloride is accelerated by increasing the temperature of the reactor.

A co-product of the halo-dehydroxylation reaction is dichlorophosphoric acid. The phosphorus oxychloride solvent is regenerated from the dichlorophophoric acid by treatment with phosphorus pentachloride. Complete recovery of the phosphporus oxychloride may be achieved typically by distillation. The resulting DCSA product may be isolated using standard distillation and filtration techniques.

Several other acids may be cyclized with the DCM-ATSA to make a substituted triazolo pyrimidine. These acids could include malonic acid, substituted malonic acid, and beta-ketocarboxylic acid or substituted carboxylic acid. However to make the DHSA or HCSA, malonic acid is used.

The above described process has several advantages over other processes, which include increased yields, nearly complete elimination of phosphorus waste and complete recovery of the phosphorus oxychloride solvent. Another advantage is that, in a preferred embodiment of the invention, solid phosphorus pentachloride is not used, but is generated in solution with phosphorus trichloride and chlorine gas. Solid phosphorus pentachloride rapidly decomposes in air and tends to agglomerate such that it is difficult to handle. Solid phosphorus pentachloride is also very corrosive. Therefore, it is highly desirable to develop a process where solid phosphorus pentachloride need not be used.

### Brief Description of the Drawings

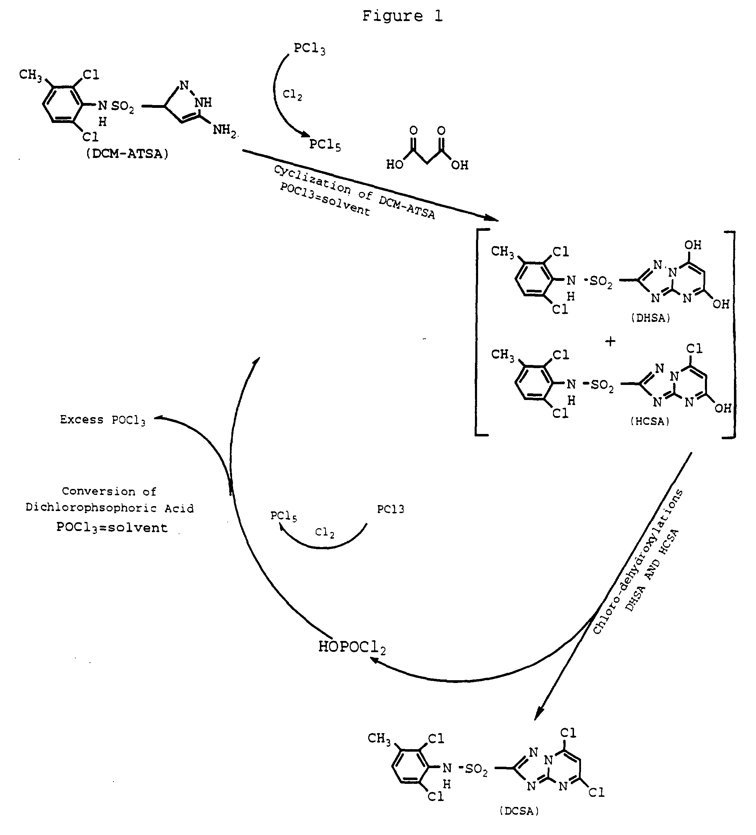

Figure 1 is a reaction schematic of one of the preferred embodiments of the invention, where the acid employed in the cyclization is malonic and the solvent is phosphorus oxychloride.

### Detailed Description of the Invention

The invention relates to an improved process for making N-(2,6-dichloro-3-methylphenyl), (4-bromo-2,6 dichloro-3-methylphenyl), (2,6-dibromo-3-methylphenyl), (2,4-dibromo-3-methylphenyl), (4,6-dibromo-3-methylphenyl), or (4-bromo-3-methylphenyl),-5-7-dihalo[1,2,4]triazolo[1,5a]pyrimidine-2-sulfonamide (which all are hereinafter referred to as DCSA) by cool cyclization of N-(2,6-dichloro-3-methylphenyl), (4-bromo-2,6 dichloro-3-methylphenyl), (2,6-dibromo-3-methylphenyl), (2,4-dibromo-3-methylphenyl), (4,6-dibromo-3-methylphenyl), or (4-bromo-3-methylphenyl),-5-amino[1,2,4]triazole-3-sulfonamide (which are all hereinafter referred to as DCM-ATSA) with an acid and phosphorus pentachloride to produce N-(2,6-dichloro-3-methylphenyl), (4-bromo-2,6 dichloro-3-methylphenyl), (2,6-dibromo-3-methylphenyl), (2,4-dibromo-3-methylphenyl), (4,6-dibromo-3-methylphenyl), or (4-bromo-3-methylphenyl),-5,7-dihydroxy[1,2,4]triazolo[1,5a]pyrimidine-2-sulfonamide (which are all hereinafter referred to as DHSA) and two isomeric compounds which are N-(2,6-dichloro-3-methylphenyl), (4-bromo-2,6 dichloro-3-methylphenyl), (2,6-dibromo-3-methylphenyl), (2,4-dibromo-3-methylphenyl), (4,6-dibromo-3-methylphenyl), or (4-bromo-3-methylphenyl),-5-hydroxy-7-chloro[1,2,4]triazolo[1,5a]pyrimidine-2-sulfonamide and N-(2,6-dichloro-3-methylphenyl), (4-bromo-2,6 dichloro-3-methylphenyl), (2,6-dibromo-3-methylphenyl), (2,4-dibromo-3-methylphenyl), (4,6-dibromo-3-methylphenyl), or (4-bromo-3-methylphenyl),-5-chloro-7-hydroxy[1,2,4]triazolo[1,5a]pyrimidine-2-sulfonamide (which are all hereinafter after referred to as HCSA). The resulting intermediates from the cyclization are then converted to DCSA by halodehydroxylation.

To make DBSA, which would be structurally the same as DCSA except that the pyrimidine portion of the molecule would be substituted with bromine instead of chlorine, the conditions and process for making DCSA are followed; however the liquid bromide, phosphorus bromide, phosphorus pentabromide and phosphorus oxybromide are used. Typically the process would comprise cyclizing DBM-ATSA with an acid selected from the group consisting of malonic and substituted malonic acid in the presence of phosphorus pentabromide and a phosphorus oxybromide at a temperature between 0°C to 60°C to produce DHSA and DBSA intermediates. After cyclization is complete, the intermediates are bromo-dehydroxylated to form a DBSA product.

Prior to cyclization of DCM-ATSA, phosphorus pentachloride is made in-situ. Phosphorus pentachloride is made by reacting phosphorus trichloride and chlorine gas. If desired, phosphorus pentachloride may be purchased instead of being made in-situ and is available from Twin Lakes Chemical in Lockport, New York.

It is preferred to form the phosphorus pentachloride prior to adding the other reactants. To form phosphorus pentachloride, between 0.1 to 5 mole-equivalents of chlorine gas, where hereinafter one mole is equivalent to one mole of DCM-ATSA, is added slowly to between 0.1 to 5 mole-equivalents of phosphorus trichloride. Preferably, between 1.5 to 2.5 mole-equivalents of chlorine gas is reacted with between 1.5 to 2.5 mole-equivalents of phosphorus trichloride, most preferably 2.2 mole-equivalents of phosphorus trichloride is used.

The reaction between chlorine gas and phosphorus trichloride is exothermic, so the reaction temperature is typically maintained between -20°C to 80°C, preferably between 0° to 50°C. The temperature is controlled by a heat sink or by adjusting the chlorine addition rate. To control the temperature within the ranges described herein, typically chlorine is added in an amount between 1.5 to 2.5 mole-equivalents in 2 to 4 hours. Preferably, prior to charging the chlorine gas through the reactor, a solvent is added to the reactor. The solvent may be phosphorus oxybromide, or phosphorus oxychloride. Other co-solvents may be used if so desired and could include chlorotoluene, methylbenzoate, methyl pivalate, chlorobenzene or alkyl benzenes, or acetonitrile. If phosphorus oxychloride is the solvent, it is used in an amount between 4 mole-equivalents to 100 mole equivalents. Typically 10 mole-equivalents of phosphorus oxychloride is used. When cosolvents are employed, the phosphorus oxychloride is typically used in an amount of 2 or greater mole-equivalents. If a co-solvent is used, the phosphorus oxychloride may be added any time prior to the halo-dehydroxylation reaction.

Upon forming the phosphorus pentachloride, the temperature of the mixture is preferably adjusted to between 0°C to 60°C, more preferably between 0°C to 20°C. Typically, the temperature is less than 10°C. After the acid is added to the reactor, the reactor is adjusted to a temperature between 10°C to 100°C, preferably betweeen 10°C to 30°C. The most preferred temperature is 20°C

After the phosphorus pentachloride is formed, the reactor is charged with DCM-ATSA and an acid. One mole equivalent of DCM-ATSA is added. All reagents are based on one mole equivalent of DCM-ATSA. Typically, between 0.5 to 5 mole-equivalent of the acid is added. Preferably, between 0.8 to 1.2 mole-equivalents per one mole of DCM-ATSA is used. The acid is selected from the group consisting of malonic acid, substituted malonic acid, where the substitution is methyl or phenyl, beta-ketocarboxylic acid, and substituted carboxylic acid. The substituted carboxylic acid is selected from the group consisting of cyanoacetic acid, aceto acetic acid, methyl malonic acid, malonic nitrile acid and phenyl malonic acid or salts thereof. Preferably, lithum, sodium or potassium salts are used.

It is thought that DCM-ATSA reacts with the phosphorus pentachloride to produce a DCM-ATSA-PCl₄ adduct and hydrochloric acid, and that the phosphorus pentachloride also reacts with the acid to produce an acid intermediate and hydrochloric acid. It is further thought that the DCM-ATSA-PCl₄ adduct is cyclized with the acid intermediate. Preferably, although not essential, DCM-ATSA is added to the reactor prior to the addition of the acid intermediate. Because the additions are exothermic, the temperature may be controlled by adjusting the rate that the DCM-ATSA and acid are added to the reactor. The temperature in the reactor during the addition is typically maintained between 0° to 60°C, preferably between 0° to 20°C, most preferably the temperature is maintained below 10°C. The cooler temperatures assist in minimizing acid decomposition and in keeping the hydrochloric acid in solution. After the acid is added to the reactor, the reactor is adjusted to a temperature between 10°C to 100°C, typically between 10°C to 50°C, preferably between 10°C to 30°C. Most preferred the temperature is 20°C. The DCM-ATSA-PCl₄ intermediate is cyclized with the acid intermediate to produce DHSA and HCSA. Typically, cyclization is complete between 4 to 40 hours, preferably between 12 to 24 hours at 20°C.

The ratio of the resulting intermediates is dependent upon the temperature of the cyclization, the concentration of the reactants and the amount of hydrochloric acid present in the mixture. Typically the ratio of intermediates is between 3:1 to 1:3 DHSA to HCSA. After cyclization, halo-dehydroxylation is accelerated by increasing the temperature of the reactor. The temperature is increased to between 30°C to 105°C, preferably between 75°C to 95°C to increase the rate of halo-dehydroxylation. It should be noted that during the cool cyclization it is thought that there might be a small amount of halo-dehydroxylation occurring. When the temperature is increased, the rate of the halo-dehydroxylation increases.

A co-product of the halo-dehydroxylation reaction is dichlorophosphoric acid or HOPOCl₂. Dihalophosphoric acid is a co-product that represents a potentially large phosphorus waste and it is thought to affect the halo-dehydroxylation rate. As the dihalophosphoric acid concentration increases, the reaction rate appears to decrease. It is thought that the phosphorus pentachloride may function as a reactant to halogenate the dihalophosphoric acid and convert the dihalophosphoric acid back to phosphorus oxychloride. The conversion may be completed by the second in-situ formation of phosphorus pentachloride either during or after the halo-dehydroxylation reaction. It is preferred to add between greater than 0 to 2.5 mole equivalents of phosphorus pentachloride. The phosphorus pentachloride may be added after the temperature is increased to accelerate the halo-dehydroxylation reaction. The phosphorus pentachloride is preferably made in-situ as discussed previously and is made once the halo-dehydroxylation temperature reaches 80°C.

By adding the phosphorus pentachloride during the halo-dehydroxylation reaction, it is thought that the conversion time of intermediates HCSA and DHSA to DCSA is decreased from between 45 hours to 20 hours. The halo-dehydroxylation conversion rate of DHSA and HCSA to DCSA may also be increased by the addition of chloride ion catalysts. These catalysts may be selected from the group consisting of lithum chloride, sodium chloride, potassium chloride, calcium chloride, tetraalkylammonium chloride, such as tetramethylammonium chloride, pyridinium-hydrochloride and the like. Conversion of DHSA and HCSA to DCSA usually takes between 20 to 60 hours, but typically between 35 to 50 hours. Generally, the conversion of the intermediates to the final product is greater than 95 percent.

The recovery of the solvent may be initiated either during or after the halo-dehydroxylation reaction. More preferably, the solvent is recovered under partial vacuum. The solvent may be distilled off and recycled as the initial loading for the next cycle of reactions. A flux solvent that has a boiling point higher than the boiling point of phosphorus oxychloride, that is non-reactive with the DCSA product may be used. The flux solvent does not have to be miscible with phosphorus oxychloride. The flux solvent is selected from the group consisting of halobenzene, alkylbenzene and substituted benzene. Preferably the flux solvent is alkylbenzene. Other flux solvents that could be used and may be selected from xylene, dichlorobenzene, chlorobenzene, nitrobenzene, chlorotoluene. anisole and the like. Most preferably, the flux solvent is xylene. The flux solvent is used to recover the remaining solvent that was not removed in the initial distillation. Typically between 5 to 25 equivalents of the solvent are recovered. Another distillation method may also be completed for total recovery of the solvent.

After halo-dehydroxylation and recovery of the solvent, the reaction solution is cooled to a temperature between 10°C to 70°C, preferably between 20°C to 50°C. A recovery solvent such as methanol, ethanol, propanol or butanol may be added to the reaction solution, which contains the flux solvent and DOSA. Any recovery solvent may be used provided that the solvent improves the solubility of the side products and lowers the solubility of the DCSA so that the DCSA may be separated from the reaction solution by filtration. It should be noted that the phosphorus oxychloride may also be recovered after the point of filtration of the DCSA product. After filtering out the DCSA, xylene could be added to the residual mixture and then the phosphorus oxychloride could be distilled off.

Preferably, the flux solvent used in the recovery of the solvent is xylene and the recovery solvent used to help disolve unwanted side products out of the resulting DCSA product is methanol. It is thought that the combination of xylene and methanol lowers the DCSA solubility, yet dissolves any impurities that may be present in the reaction mixture. After the recovery solvent is added, typically the reaction solution is maintained at a temperature between 0°C to 70°C for between greater than 0 to 100 hours, preferably the reaction solution is maintained at a temperature between 20°C to 50°C for greater than 0 to 2 hours. The resulting slurry is filtered to isolate the DCSA.

Figure 1 is an illustration of one of the preferred embodiments of the invention. The phosphorus pentachloride is made in-situ and the DCM-ATSA and malonic acid are added to a reactor along with phosphorus oxychloride. After the cyclization is complete, the resulting intermediates, DHSA and HCSA, undergo a chloro-dehydroxylation reaction to produce the final product DCSA. The co-product dichlorophosphoric acid may be converted back to the solvent phosphorus oxychloride with phosphorus pentachloride.

### Examples

The following examples outline general procedures and operating conditions which may be used to produce DCSA. The examples are presented to illustrate the invention.

### Example 1

Example 1 consists of three operations: reactions, distillation, and DCSA isolation. The scale of the process was 0.8 mole of DCM-ATSA, and one mole-equivalent, therefore, equals 0.8 mole.

### Part A- Reaction

### Cyclization and Halo-dehydroxylation

A 2-liter reactor was purged with nitrogen and charged with POCl₃, 10 mole-equivalents/mole of DCM-ATSA and PCl₃, and 2.2 mole-equivalents/mole DCM-ATSA. The reactor was equipped with an air-driven, overhead stirrer and thermowell. The kettle top had a total of five necks. A circulating bach fed and controlled the temperature of the DOWTHERM LF™ in the jacket of this reactor. The reactor was vented through a dry-ice condenser, a 1 liter knock-out flask and a 1 liter caustic trap. A mineral oil bubbler was placed after the knock-out flask to prevent water vapor from reaching the reactor. Both POCl₃ (10 mole equivalents) and PCl₃ (2.2 mole equivalents) are liquids that were added to the reactor at 20°C. Through a 0.5-inch (1.25 cm) teflon addition tube, chlorine gas (2.2 mole-equivalents/mole of DCM-ATSA) was slowly added over approximately 1.5 hours. The reaction cf chlorine gas with PCl₃ was exothermic. The temperature of the contents were kept below 30°C. Because the product of this reaction, PCl₅, was a solid and not very soluble in POCl₃ at this temperature, the mixture became a slurry. Slight nitrogen pressure was sometimes kept with the Cl₂ to prevent plugging in the addition tube. A dry-ice condenser was used to minimize chlorine loss. After the chlorine charge, the addition tube was removed, and a stopper was put in its place. The reactor contents were then cooled to 6°C. The reactor was charged with two solids: 1 mole-equivalent of DCM-ATSA and 1.02 mole-equivalents of malonic acid. Because these additions were exochermic, the rates were adjusted to keep the slurry temperature below 10°C. This cold addition kept HCl in solution and minimized malonic acid decomposition. The reactor contents were then warmed to 20 °C. After 13 to 15 hours at this temperature, the cyclization of the DCM-ATSA/PCl₄ adduce was considered complete. After the cyclization of DCM-ATSA/PCl₄ adduct, the rate of the chloro-dehydroxylation of DHSA and HCSA with POCl₃ was increased by raising the reactor temperature to 80°C. Sufficient conversion to DCSA required 40 hours at this temperature.

The slurry was cooled to approximately 25°C before the reactor was charged with PCl₃, and 4 mole equivalents/mole of DCM-ATSA. Through the same 0.5-inch teflon addition cube, 2.1 mole-equivalents of chlorine were again added slowly (over approximately 1.5 hours) to the reactor. The PCl₅ reacted with the dichlorophosphoric acid to produce POCl₃. The temperature of the slurry was kept below 80°C. After the chlorine charge, the addition tube was removed, and a scopper was put in its place.

### Part B- Distillation

The equipment was then prepared for vacuum distillation to recover the POCl₃. The dry-ice condenser was replaced with a distillation column on the kettle top. This vacuum-jacketed and silvered column had a 1-inch (2.5 cm) internal diameter and a 32-inch (81 cm) packing height. The packing was 1/8 inch (0.32 cm) glass helices. A second circulation bath was used to feed the DOWTHERM LF™ to the condenser at 6°C. An acetone/dry-ice bath was also used to cool the overhead receiving flask to reduce the vapor pressure of the collected liquid. Vacuum was achieved with a belt-driven, single-stage pump. The system pressure was controlled with a vacuum regulator and measured with a mercury manometer. The pump, manometer and reaulator were protected by two dry-ice traps.

An absolute pressure of about 250 mmHg (0.333 bar) was achieved. This reduced pressure allowed the distillation to run at lower temperatures which minimized DCSA decomposition. The jacket temperature was then raised to 100°C to begin the distillation. The temperature drop from the jacket to the reactor contents was normally 20°C during the distillation. The reflux ratio was controlled with a timer, electromagnetic coil and magnetic distilling head. The first overhead cut was collected with a constant reflux ratio of 2. This overhead cut consisted of approximately 2 mole-equivalents of PCl₃ and 10 mole-equivalents of POCl₃. This material was stored in a tightly-capped bottle for recycle to the next batch run as the initial solvent charged in the beginning of the reaction.

To facilitate the recovery of the remaining solvent, the reflux ratio was increased to 6. The jacket temperature and absolute pressure were 100°C and approximately 250 mmHg (0.333 bar), respectively. The temperature drop from the jacket to the reactor contents was 20°C. This second overhead cut was about 4 mole-equivalents of POCl₃, which was the amount generated from PCl₅ during the cyclization of DCM-ATSA and conversion of dichlorophosphoric acid. A mixture of 4 mole-equivalents of POCl₃ and 1.5 mole-equivalents of o-xylene was charged to the reactor during the collection of this second distillate through the addition funnel. A nitrogen blanket was kept above the liquid addition funnel. This feed allowed the recovered POCl₃ to be of high purity. The feed was normally charged continuously over three of the five hours needed for collection. The recovered POCl₃ was stored in a separate, tightly capped bottle.

The third overhead cut had a composition approximately equal to the material fed during the collection of the second overhead cut described above. This final distillate, therefore, was intended for recycle to the next batch run distillation. The absolute pressure, reflux ratio and jacket temperature were 80 mmHg (0.107 bar), 5, and 100°C, respectively, during the collection. The temperature drop from the jacket to the reactor contents was normally 20°C. The reactor was charged continuously with 7 mole-equivalents of o-xylene over four of the six hours needed for collection. This distillate was stored in a separate. tightly capped bottle.

### Part C- DCSA Isolation

After the distillation, the reactor contained a slurry of o-xylene, DCSA, some side products, and traces of phosphorus oxychloride. The distillation column was removed from the reactor to prevent contamination. When the reactor contents were at ambient pressure and 50°C, 7 to 21 mole-equivalents of methanol were added to help dissolve the side products. The slurry was then kept at 50°C for approximately one hour.

The reactor contents were cooled to 25°C, drained, and filtered. The resulting solid was washed with 2 to 3 cake volumes of methanol. The density of the wet cake was about 1.0 g/mL. The mother-liquor/filtrate mixture was stored in a tightly capped bottle. A piece of latex was then placed over the cake and vacuum maintained to remove excess solvent.

### Example 2

The procedures described in Example 1 were followed, except that the reaction conditions for trials 1 through 15 varied according to the conditions specified in Table 1 and the DCSA was isolated prior to the phosphorus oxychloride recovery.

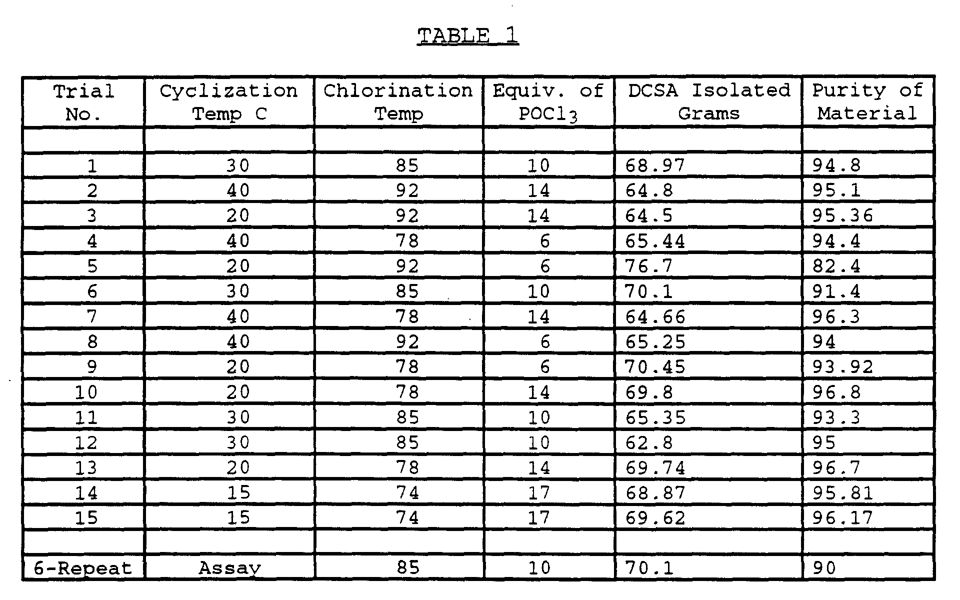

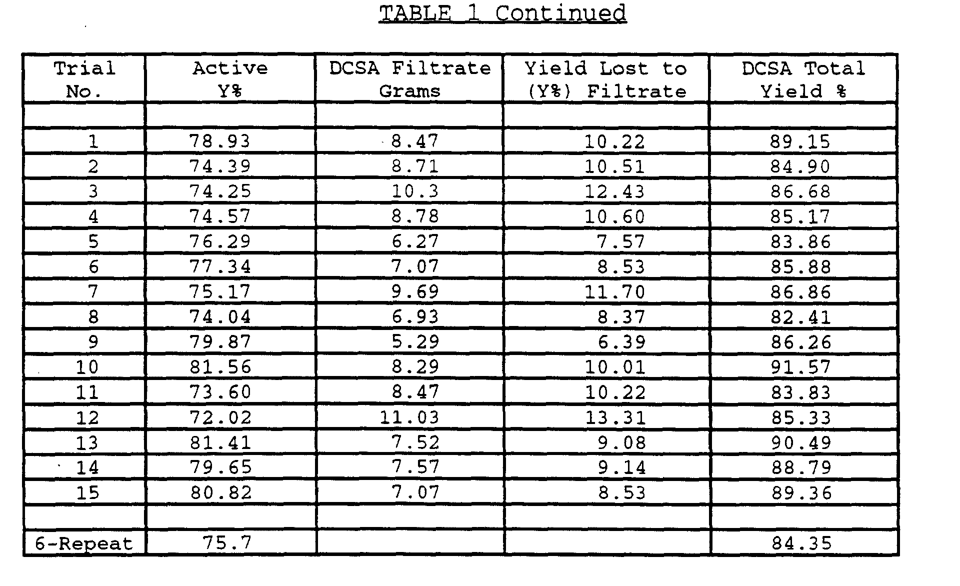

As can be seen from Table 1, the range of DCSA conversion was between 82 to 91 percent with an isolated active yield range of between 72 to 82 percent.

## Claims

1. A process for preparing a compound of formula I ("DCSA") 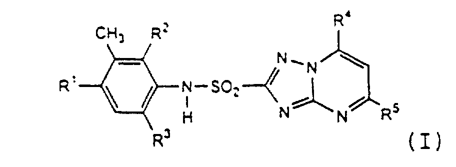 where R⁴ and R⁵ are both Cl or both Br, and the values of R¹, R² and R³ have one of the following combinations:
| R¹ | R² | R³ |
|---|---|---|
| H | Cl | Cl |
| Br | Cl | Cl |
| H | Br | Br |
| Br | Br | H |
| Br | H | Br |
| Br | H | H |
which comprises
combining a compound of the formula II ("DCM-ATSA") 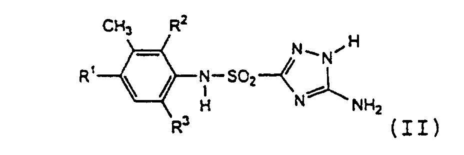 wherein R¹, R², and R³ are as defined in formula I, with malonic acid, in the presence of either phosphorus pentachloride and a phosphourus oxychloride solvent when a product wherein R⁴ and R⁵ are Cl is desired or phosphorus pentabromide and a phosphourus oxybromide solvent when a product wherein R⁴ and R⁵ are Br is desired, at a temperature between 0°C to 60°C, to form a phosphorus tetrachloride or tetrabromide adduct of the compound of formula II, and
cyclising the phosphorus tetrachloride or tetrabromide adduct of the compound of formula II with the malonic acid, to produce a mixture of a compound of formula III ("DHSA") 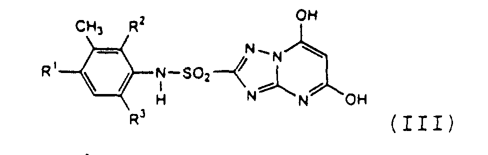 wherein R¹, R², and R³ are as defined in formula I, and compounds of formulas IVa and IVb ("HCSA") 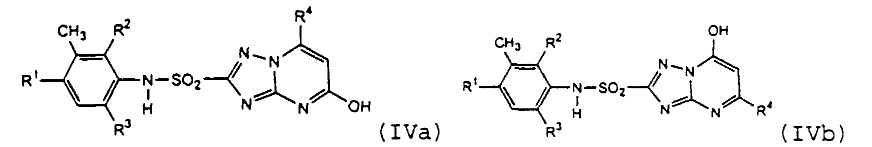 wherein R¹, R², R³ and R⁴ are as defined in formula I, and, after cyclization is complete,
halodehydroxylating the mixture of compounds of formulas III, IVa and IVb to form the product of formula I.

2. The process of claim 1 wherein R⁴ and R⁵ are both Cl and prior to cyclization said phosphorus pentachloride is made in situ by reacting chlorine gas and phosphorus trichloride.

3. The process of claim 1 wherein R⁴ and R⁵ are both Cl and the amount of phosphorus pentachloride is between 2 to 2.5 mole equivalents per mole of compound of formula II ("DCM-ATSA").

4. The process of claim 1 wherein between 0.5 to 5 mole equivalents of malonic acid is used per mole of compound of formula II ("DCM-ATSA").

5. The process of claim 4 wherein between 0.8 to 1.2 mole equivalents of malonic acid is used per mole of compound of formula III ("DCM-ATSA").

6. The process of claim 1 wherein the reaction mixture is maintained at a temperature of between 10°C to 100°C during the cyclization step.

7. The process of claim 6 wherein the reaction mixture is maintained at a temperature of between 10°C to 30°C during the cyclization step.

8. The process of claim 7 wherein the reaction mixture is maintained at a temperature of 20°C during the cyclization step.

9. The process of claim 1 wherein the temperature of the reaction mixture is between 30°C to 105°C during the halodehydroxylation step.

10. The process of claim 9 wherein the temperature of the reaction mixture is between 75°C to 95°C during the halodehydroxylation step.

11. The process of claim 1 wherein R⁴ and R⁵ are both Cl and dichloro-phosphoric acid is a coproduct of the halodehydroxylation.

12. The process of claim 11 wherein phosphorus pentachloride reacts with the dichloro-phosphoric acid to make phosphorus oxychloride.

13. The process of claim 12 wherein between greater than 0 to 2.5 mole equivalents of phosphorus pentachloride is added to the reaction mixture during the halodehydroxylation.

14. The process of claim 1 wherein the solvent is recovered by distillation and a flux solvent is added during distillation, where the resulting reaction mixture recovered contains a compound of formula I ("DCSA") and a flux solvent.

15. The process of claim 14 wherein the flux solvent is substituted benzene.

16. The process of claim 15 wherein the flux solvent is xylene, dichlorobenzene, nitrobenzene, chorotoluene, or anisole.

17. The process of claim 15 wherein a recovery solvent is added after distillation to the resulting mixture and the recovery solvent is methanol, ethanol, propanol, or butanol.

18. The process of claim 1 wherein R⁴ and R⁵ are both C1 and the rate of conversion of compound of formula II ("DCM-ATSA") to the compound of formula I ("DCSA") is increased by the addition of chloride ion catalysts.

19. The process of claim 18 wherein the chloride ion catalyst is selected from the group consisting of lithium chloride, sodium chloride, potassium chloride, calcium chloride, tetraalkylammonium chloride, and pyridinium hydrochloride.

20. The process of claim 19 wherein the tetraalkylammonium chloride is tetramethylammonium chloride.

21. The proces of claim 1 wherein a co-solvent is used with the phosphorus oxychloride and is selected from the group consisting of chlorotoluene, methylbenzoate, methylpivalate chlorobenzene, alkylbenzene, and acetonitrile.

22. The process of claim 1 wherein R⁴ and R⁵ are both Cl, and the halodehyroxylation step is chlorodehydroxylation.

## Patentansprüche

1. Verfahren zur Herstellung einer Verbindung nach Formel I ("DCSA") 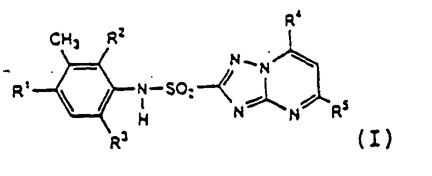 wobei R⁴ und R⁵ entweder beide Cl oder beide Br sind, und die Bedeutungen von R¹, R² und R³ eine der folgenden Kombinationen sind:
| R¹ | R² | R³ |
|---|---|---|
| H | Cl | Cl |
| Br | Cl | Cl |
| H | Br | Br |
| Br | Br | H |
| Br | H | Br |
| Br | H | H |
umfassend
Kombinieren einer Verbindung nach Formel II ("DCM-ATSA"), 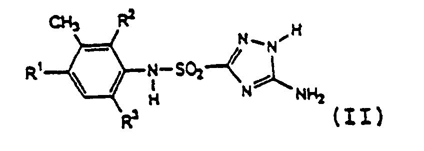 wobei R¹, R² und R³ wie in Formel I definiert sind, mit Malonsäure, in der Gegenwart von entweder Phosphorpentachlorid und einem Phosphoroxychlorid-Lösungsmittel, wenn ein Produkt gewünscht wird, bei dem R⁴ und R⁵ Cl sind, oder Phosphorpentabromid und einem Phosphoroxybromid-Lösungsmittel, wenn ein Produkt gewünscht wird, bei dem R⁴ und R⁵ Br sind, bei einer Temperatur zwischen 0°C und 60°C, wobei ein Phosphortetrachlorid- oder Phosphortetrabromid-Addukt der Verbindung nach Formel 11 gebildet wird, und
Zyklisieren des Phosphortetrachlorid- oder Phosphortetrabromid-Addukts der Verbindung nach Formel II mit der Malonsäure, wobei ein Gemisch aus einer Verbindung nach Formel III ("DHSA") 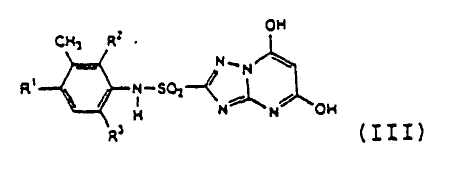 wobei R¹, R² und R³ wie in Formel 1 definiert sind, und von Verbindungen nach den Formeln IVa und IVb ("HCSA") 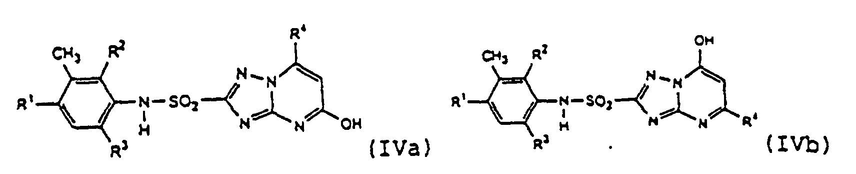 wobei R¹, R² und R³ wie in Formel I definiert sind, erzeugt wird, und nach vollständiger Zyklisierung,
Halodehydroxylieren des Gemisches von Verbindungen nach den Formeln III, IVa und IVb, wobei das Produkt nach Formel I gebildet wird.

2. Verfahren nach Anspruch 1, wobei R⁴ und R⁵ beide CI sind und vor der Zyklisierung das Phosphorpentachlorid in situ durch Reaktion von Chlorgas und Phosphortrichlorid hergestellt wird.

3. Verfahren nach Anspruch 1, wobei R⁴ und R⁵ beide Cl sind und die Menge an Phosphorpentachlorid zwischen 2 und 2,5 Moläquivalente pro Mol Verbindung nach Formel II ("DCM-ATSA") beträgt.

4. Verfahren nach Anspruch 1, wobei zwischen 0,5 und 5 Moläquivalente Malonsäure pro Mol Verbindung nach Formel II ("DCM-ATSA") verwendet werden.

5. Verfahren nach Anspruch 4, wobei zwischen 0,8 und 1,2 Moläquivalente Malonsäure pro Mol Verbindung nach Formel II ("DCM-ATSA") verwendet werden.

6. Verfahren nach Anspruch 1, wobei das Reaktionsgemisch während des Zyklisierungsschritts bei einer Temperatur zwischen 10°C und 100°C gehalten wird.

7. Verfahren nach Anspruch 6, wobei das Reaktionsgemisch während des Zyklisierungsschritts bei einer Temperatur zwischen 10°C und 30°C gehalten wird.

8. Verfahren nach Anspruch 7, wobei das Reaktionsgemisch während des Zyklisierungsschritts bei einer Temperatur von 20°C gehalten wird.

9. Verfahren nach Anspruch 1, wobei die Temperatur des Reaktionsgemisches während des Halodehydroxylierungsschritts zwischen 30°C und 105°C gehalten wird.

10. Verfahren nach Anspruch 9, wobei die Temperatur des Reaktionsgemisches während des Halodehydroxylierungsschritts zwischen 75°C und 95°C gehalten wird.

11. Verfahren nach Anspruch 1, wobei R⁴ und R⁵ beide Cl sind und Dichlorphosphorsäure ein Nebenprodukt der Halodehydroxylierung ist.

12. Verfahren nach Anspruch 11, wobei Phosphorpentachlorid mit der Dichlorphosphorsäure reagiert, wobei Phosphoroxychlorid erzeugt wird.

13. Verfahren nach Anspruch 12, wobei zwischen größer 0 und 2,5 Moläquivalente Phosphorpentachlorid während der Halodehydroxylierung zu dem Reaktionsgemisch zugegeben werden.

14. Verfahren nach Anspruch 1, wobei das Lösungsmittel mittels Destillation gewonnen wird und während der Destillation ein Flußlösungsmittel zugegeben wird, wobei das gewonnene resultierende Reaktionsgemisch eine Verbindung nach Formel I ("DCSA") und ein Flußlösungsmittel enthält.

15. Verfahren nach Anspruch 14, wobei das Flußlösungsmittel substituiertes Benzol ist.

16. Verfahren nach Anspruch 15, wobei das Flußlösungsmittel Xylol, Dichlorbenzol, Nitrobenzol, Chlortoluol oder Anisoi ist.

17. Verfahren nach Anspruch 15, wobei nach der Destillation zu dem resultierenden Gemisch ein Rückgewinnungslösungsmittel zugegeben wird, und das Rückgewinnungslösungsmittel Methanol, Ethanol, Propanol oder Butanol ist.

18. Verfahren nach Anspruch 1, wobei R⁴ und R⁵ beide Cl sind und die Geschwindigkeit der Umsetzung von Verbindung nach Formel II ("DCM-ATSA") zu der Verbindung nach Formel I ("DCSA") durch die Zugabe von Chloridionenkatalysatoren erhöht wird.

19. Verfahren nach Anspruch 18, wobei der Chloridionenkatalysator ausgewählt wird aus der Gruppe, bestehend aus Lithiumchlorid, Natriumchlorid, Kaliumchlorid, Calciumchlorid, Tetraalkylammoniumchlorid und Pyridiniumhydrochlorid.

20. Verfahren nach Anspruch 19, wobei das Tetraalkylammoniumchlorid Tetramethylammoniumchlorid ist.

21. Verfahren nach Anspruch 1, wobei ein Colösungsmittel mit dem Phosphoroxychlorid verwendet wird, und aus der Gruppe, bestehend aus Chlortoluol, Methylbenzoat, Methylpivalat, Chlorbenzol, Alkylbenzol und Acetonitril, ausgewählt wird.

22. Verfahren nach Anspruch 1, wobei R⁴ und R⁵ beide Cl sind, und der Halodehydroxylierungsschritt Chlordehydroxylierung ist.

## Revendications

1. Procédé de préparation d'un composé de formule I ("DCSA") : 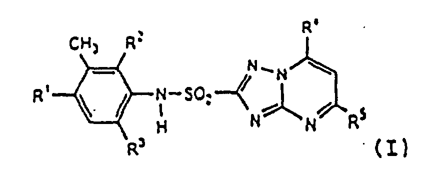 dans laquelle R⁴ et R⁵ représentent tous deux C1 ou tous deux Br, et les significations de R¹, R² et R³ correspondent à l'une quelconque des combinaisons suivantes :
| R¹ | R² | R³ |
|---|---|---|
| H | Ci | Cl |
| Br | Cl | Cl |
| H | Br | Br |
| Br | Br | H |
| Br | H | Br |
| Br | H | H |
procédé qui comprend les étapes consistant à : combiner un composé de formule II ("DCM-ATSA") : 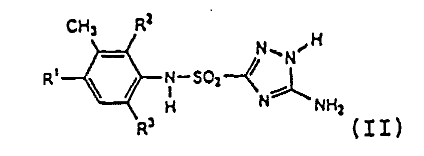 dans laquelle R¹, R² et R³ sont tels qu'ils sont définis au sujet de la formule I, avec de l'acide malonique, en présence de pentachlorure de phosphore et d'oxychlorure de phosphore comme solvant quand on souhaite obtenir un produit où R⁴ et R⁵ représentent Cl, ou de pentabromure de phosphore et d'oxybromure de phosphore comme solvant quand on souhaite obtenir un produit où R⁴ et R⁵ représentent Br, à une température comprise entre 0 °C et 60 °C, pour former un produit d'addition tétrachlorure ou tétrabromure de phosphore-composé de formule II,
cycliser le produit d'addition précédent avec l'acide malonique pour produire un mélange d'un composé de formule III ("DHSA") : 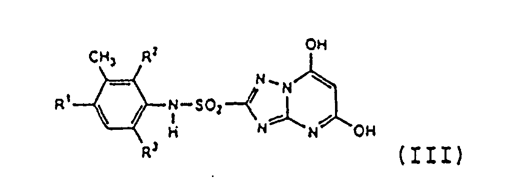 dans laquelle R¹, R² et R³ sont tels qu'ils sont définis au sujet de la formule I, et de composés de formules IVa et IVb ("HCSA") : 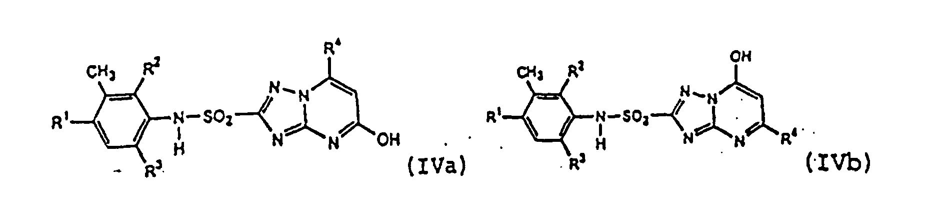 dans lesquelles R¹, R², R³ et R⁴ sont tels qu'ils sont définis au sujet de la formule I, et une fois terminée la cyclisation, déshydroxyler et halogéner le mélange des composés de formules III, IVa et IVb pour former le produit de formule I.

2. Procédé selon la revendication 1, dans lequel R⁴ et R⁵ représentent tous deux Cl et, avant la cyclisation, on produit ledit pentachlorure de phosphore in situ par réaction de chlore gazeux avec du trichlorure de phosphore.

3. Procédé selon la revendication 1, dans lequel R⁴ et R⁵ représentent tous deux Cl et la quantité de pentachlorure de phosphore est de 2 à 2,5 équivalents molaires par mole de composé de formule II ("DCM-ATSA").

4. Procédé selon la revendication 1, dans lequel on utilise 0,5 à 5 équivalents molaires d'acide malonique par mole de composé de formule II ("DCM-ATSA").

5. Procédé selon la revendication 4, dans lequel on utilise 0,8 à 1,2 équivalent molaire d'acide malonique par mole de composé de formule II ("DCM-ATSA").

6. Procédé selon la revendication 1, dans lequel on maintient le mélange réactionnel à une température comprise entre 10 °C et 100 °C pendant l'étape de cyclisation.

7. Procédé selon la revendication 6, dans lequel on maintient le mélange réactionnel à une température comprise entre 10 °C et 30 °C pendant l'étape de cyclisation

8. Procédé selon la revendication 7, dans lequel on maintient le mélange réactionnel à une température de 20 °C pendant l'étape de cyclisation.

9. Procédé selon la revendication 1, dans lequel la température du mélange réactionnel est comprise entre 30 °C et 105 °C pendant l'étape de déshydroxylation et d'halogénation.

10. Procédé selon la revendication 9, dans lequel la température du mélange réactionnel est comprise entre 75 °C et 95 °C pendant l'étape de déshydroxylation et d'halogénation.

11. Procédé selon la revendication 1, dans lequel R⁴ et R⁵ sont tous deux Cl et il se forme de l'acide dichlorophosphorique comme sous-produit lors de l'étape de déshydroxylation et d'halogénation.

12. Procédé selon la revendication 11, dans lequel on fait réagir du pentachlorure de phosphore avec l'acide dichlorophosphorique pour produire de l'oxychlorure de phosphore.

13. Procédé selon la revendication 12, dans lequel on ajoute au mélange réactionnel, pendant l'étape de déshydroxylation et d'halogénation, une quantité de pentachlorure de phosphore comprise entre plus de O et 2,5 équivalents molaires.

14. Procédé selon la revendication 1, dans lequel on récupère le solvant par distillation et on ajoute lors de la distillation un solvant d'entraînement, de sorte que le mélange réactionnel résultant récupéré contient un composé de formule I ("DCSA") et un solvant d'entraînement.

15. Procédé selon la revendication 14, dans lequel le solvant d'entraînement est un dérivé substitué du benzène.

16. Procédé selon la revendication 15, dans lequel le solvant d'entraînement est le xylène, un dichlorobenzène, le nitrobenzène, un chlorotoluène ou l'anisole.

17. Procédé selon la revendication 15, dans lequel, après la distillation, on ajoute au mélange résultant un solvant de récupération et le solvant de récupération est le méthanol, l'éthanol, le propanol ou le butanol.

18. Procédé selon la revendication 1, dans lequel R⁴ et R⁵ sont tous deux Cl et on augmente la vitesse de transformation du composé de formule II ("DCM-ATSA") en le composé de formule I ("DCSA") en ajoutant des ions chlorure comme catalyseur.

19. Procédé selon la revendication 18, dans lequel le catalyseur fournissant des ions chlorure est choisi parmi le chlorure de lithium, le chlorure de sodium, le chlorure de potassium, le chlorure de calcium, les chlorures de tétraalkylammonium et le chlorhydrate de pyridinium.

20. Procédé selon la revendication 19, dans lequel le chlorure de tétraalkylammonium est le chlorure de tétraméthylammonium.

21. Procédé selon la revendication 1, dans lequel on utilise avec l'oxychlorure de phosphore un cosolvant qui est choisi parmi les chlorotoluènes, le benzoate de méthyle, le pivalate de méthyle, le chlorobenzène, les alkylbenzènes et l'acétonitrile.

22. Procédé selon la revendication 1, dans lequel R⁴ et R⁵ représentent tous deux Cl et l'étape de déshydroxylation et d'halogénation est une étape de déshydroxylation et de chloration.
